# EUROPEAN PATENT SPECIFICATION

(11) **EP 2 493 456 B1**
(45) Date of publication and mention of the grant of the patent: **21.11.2018**
(21) Application number: 10790593.7
(22) Date of filing: 27.10.2010
(51) Int. Cl.: A61K 9/20, A61K 9/14, A61K 31/437

(54) **SOLID DISPERSION OF RIFAXIMIN**
FESTSTOFFDISPERSION AUS RIFAXIMIN
DISPERSION SOLIDE DE RIFAXIMINE

(30) Priority: 27.10.2009 IN 1287KO2009
(43) Date of publication of application: 05.09.2012
(73) Proprietor: Lupin Limited, Mumbai 400 055 (IN)
(72) Inventor: KULKARNI, Shirishkumar, Pune 411 042 Maharashtra (IN); DALAL, Satish, Kumar, Pune 411 042 Maharashtra (IN); JAHAGIRDAR, Harshal, Anil, Pune 411 042 Maharashtra (IN)
(74) Representative: Harrison IP Limited
(86) International application number: PCT/IN2010/000694
(87) International publication number: WO 2011/051971

(56) References cited:
- EP-A1- 2 011 486
- WO-A1-02/051385
- WO-A1-2010/067072
- US-A- 4 341 785
- ARYA ET AL: "Rifaximin-the promising anti-microbial for enteric infections", JOURNAL OF INFECTION, ACADEMIC PRESS, LONDON, GB, vol. 51, no. 3, 1 October 2005 (2005-10-01), page 262, XP005144960, ISSN: 0163-4453, DOI: DOI:10.1016/J.JINF.2005.07.018
- VASCONCELOS ET AL: "Solid dispersions as strategy to improve oral bioavailability of poor water soluble drugs", DRUG DISCOVERY TODAY, ELSEVIER, AMSTERDAM, NL, vol. 12, no. 23-24, 30 October 2007 (2007-10-30), pages 1068-1075, XP022370275, ISSN: 1359-6446

## Description

### Field of The Invention

The present invention relates to solid dispersion of rifaximin, which increases the solubility of rifaximin and improves gastrointestinal availability. It also relates to pharmaceutical compositions comprising solid dispersion of rifaximin.

### Background of the Invention

The antibiotic rifaximin was originally disclosed in Italian Patent No. 1154655. The related U.S. Pat. No. 4,341,785 to Marchi et al. discloses imidazo-rifamycin derivatives having antibacterial utility, and the related process for preparing it. The US '785 patent also discloses a pharmaceutical antibacterial composition and a method of using it to treat antibacterial diseases of the gastrointestinal tract (GIT).

Rifaximin is essentially a non-absorbable, non-systemic, semi-synthetic antibiotic, related to rifamycin. The antimicrobial spectrum includes most gram-positive and gram-negative bacteria; and both aerobes and anaerobes. Rifaximin is approved in certain countries for the treatment of pathologies whose etiology is in part or totally due to intestinal acute and chronic infections sustained by Gram-positive and Gram-negative bacteria, with diarrhea syndromes, altered intestinal microbial flora, summer diarrhea-like episodes, traveler's diarrhea and enterocolitis, pre- and post- surgery prophylaxis of the infective complications in gastro intestinal surgery; and hyperammonaemia therapy as coadjutant.

Rifaximin is currently marketed as 200mg tablets for traveler's diarrhea under the brand name "Xifaxan®". The advantages of rifaximin to treat these infections are two-fold: (1) site-targeted antibiotic delivery; and (2) improved tolerability compared to other treatments.

Rifaximin is practically insoluble in water and is virtually undissolved in the gastro intestinal tract (GIT). The relative insolubility of rifaximin also leads to its negligible systemic absorption. Less than 0.5% of the drug is absorbed into the bloodstream when taken orally.

Rifaximin has been known to be effective for treating infections that are localized to the gut and is not known to be suitable for treating systemic infections caused by invasive organisms. By increasing the solubility of rifaximin, dose can be reduced significantly thereby making it more patient compliant. There is a need for development of rifaximin in a form which will increase its solubility and improve the gastrointestinal availability.

WO 2010067072 by Ghagare et al. discloses a rifaximin complex to increase the solubility of rifaximin thereby increasing the bioavailability of the rifaximin in the body.

EP2011486 discloses hot melt extrusion of rifaximin with polyethylene oxide and hydroxypropylmethylcellulose.

We have prepared solid dispersion of rifaximin, which surprisingly increases solubility and the gastrointestinal availability of rifaximin.

### Summary of the Invention

The object of the invention is to provide solid dispersion of rifaximin. The present invention provides a solid dispersion of rifaximin comprises rifaximin and pharmaceutically acceptable carrier wherein the pharmaceutically acceptable carrier is selected from polyethylene glycol, polymethacrylates, polyvinyl acetate, cellulose derivatives, polaxamer, glyceryl behenate, polyethyleneglycol derivative of a mono-glyceride, vitamin E, polyethylene or polyoxyethylene ester of hydroxyl stearic acid, polyoxylglycerides, polyethoxylated castor oil or combinations thereof,

In another embodiment, solid dispersion of rifaximin increases the solubility of rifaximin relative to an equivalent amount of rifaximin.

Another embodiment relates to solid dispersion of rifaximin, wherein the solubility of rifaximin is increased by more than 30%.

In another embodiment, solid dispersion of rifaximin increases the gastrointestinal availability of rifaximin relative to an equivalent amount of rifaximin.

Another embodiment relates to solid dispersion of rifaximin, wherein the solubility of rifaximin in the solid dispersion is increased relative to an equivalent amount of rifaximin, while maintaining equivalent permeability relative to an equivalent amount of rifaximin.

In another embodiment, solid dispersion of rifaximin increases both solubility and gastrointestinal availability of rifaximin. Specifically, the dispersion provides higher solubility of rifaximin and improved gastrointestinal (GIT) availability relative to an equivalent dose of rifaximin in a commercially available tablet, or to an equivalent dose of rifaximin in a simple solution such as in water or normal saline solution, that is orally ingested.

Another embodiment relates to pharmaceutical composition comprising solid dispersion of rifaximin.

Another embodiment relates to pharmaceutical composition comprising stable solid dispersion comprising therapeutically effective amount of rifaximin, which provides higher solubility of rifaximin and improved gastrointestinal (GIT) availability relative to an equivalent dose of rifaximin in a commercially available tablet, or to an equivalent dose of rifaximin in a simple solution such as in water or normal saline solution, that is orally ingested.

Another embodiment relates to the solid dispersion of rifaximin for use in treating and/or preventing a microbial infection.

Another embodiment relates to the solid dispersion of rifaximin for use in the treatment or prevention of irritable bowel syndrome or Crohn's disease.

### Detailed Description of the Invention

The present invention relates to solid dispersion of rifaximin, comprising rifaximin and pharmaceutically acceptable carrier, which increases the solubility and gastrointestinal availability of rifaximin.

It also relates to pharmaceutical composition comprising solid dispersion of rifaximin comprising therapeutically effective amount of rifaximin, which increases the solubility of rifaximin and improves gastrointestinal availability.

Technologies for increasing drug solubility include chemical modification such as prodrug or salt formation; physical modification such as solid dispersions, nanocrystals and nanoparticles, co-crystals and loading on porous structures; alteration of solvent composition such as pH adjustments, co-solvents & wetting agents; carrier systems such as cyclodextrins, inclusion complexes, liposomes, polymeric micelles, emulsions, microemulsions & ampiphilic polymers, surfactant dispersions, micronization by colloid mills or jet mills and like wise.

Solid dispersion technique has often proved to be the most commonly used technique in improving dissolution of poorly soluble active pharmaceutical ingredients because it is simple, economic and advantageous.

The advantages of solid dispersions are increased wettability due to dispersion in a hydrophilic carrier; reduced drug particle size and hence increased surface area in two-phase solid dispersions; reduced crystallinity or creation of amorphous systems.

Solid dispersion can be in single phase such as substitutional or interstitial crystalline solutions or amorphous solutions; or it can be a two-phase system such as eutectics, crystalline drug amorphous carrier or amorphous drug and amorphous carrier dispersions. Solid solutions are a resultant single phase upon dispersion of two compounds in each other, at their molecular level.

Various methods of forming solid dispersion includes traditional melt cool method, hot stage extrusion, melt agglomeration, meltrex®; coprecipitation, solvent evaporation such as vaccum drying, hot plate drying, slow evaporation at low temperature, rotary evaporation, spray drying, freeze drying, spin drying and super critical fluid drying.

### Definitions:

As the term is used herein, "rifaximin" refers to rifaximin base, pharmaceutically acceptable salts, polymorph(s), solvate(s), hydrate(s), enantiomer(s) thereof unless otherwise specified.

"Therapeutically effective amount" means that the amount of active agent, which halts or reduces the progress of the condition being treated or which otherwise completely or partly cures or acts palliatively on the condition. A person skilled in the art can easily determine such an amount by routine experimentation without undue burden.

The term "solid dispersion" means the finely dispersed distribution of one or more components, e.g. an active substance like rifaximin, in an inert solid or semi-solid carrier which increases wettability and/ or solubility; it also embraces semi-solid dispersions. The active substance may be present in molecular dispersed form, i.e. as a solid solution, in fine crystalline dispersed form, in a glassy amorphous phase or dispersed as a fine amorphous powder. Eutectic mixtures, i.e. crystalline structures of active substances and carriers are also encompassed in the definition of "solid dispersions".

"Solubility" means solubility of rifaximin in aqueous media such as water, buffer, gastrointestinal simulated fluid, gastrointestinal fluid and the like.

A "formulation" or "composition" as the term is used herein is a composition of matter including rifaximin and other components, such as excipients, stabilizers, dispersants, surfactants, and the like.

By "pharmaceutically acceptable" is meant a carrier comprised of a material that is not biologically or otherwise undesirable.

"Administering" or "administration" refers to providing a medicinal compound to a patient in need thereof.

A "frequency" of administration refers to how often the medication is given when repeated doses are prescribed; for example, the medication can be administered daily. "Duration" refers to the period of time over which repeated doses are administered.

"Modified release," means drug delivery system releasing the drug at a predetermined rate, locally or systemically, for a specified period of time. In other words, modified release is other than immediate release. Modified release can be used interchangeably with prolonged release, programmed release, timed release, extended release, sustained release, controlled release, delayed release, pulsatile release and other such dosage forms.

A solid dispersion of rifaximin comprises rifaximin and pharmaceutically acceptable carrier. It further comprises surfactants, emulsifiers, stabilizing agents etc. Solvent is used in the preparation of solid dispersion which is to be removed. Rifaximin and the pharmaceutically acceptable carrier can be present in various ratios.

The carrier and the solvent selection depend upon the chemistry of rifaximin. The most relevant selection criteria are miscibility of the carriers and good stability during the solid dispersion manufacturing process and storage thereupon.

Pharmaceutically acceptable carriers used to prepare solid dispersion of rifaximin include polyethylene glycol, povidone, copovidone, polymethacrylates, polyvinyl acetate, cellulose derivatives, self emulsifying carriers, poloxamer, glyceryl behenate (Compritol), gelucire (polyethyleneglycol derivative of a mono-glyceride, i.e. a PEG-ylated monoglyceride), vitamin E such as tocophersol, tocotrienols; polyethylene or polyoxyethylene ester of hydroxyl stearic acid such as solutol HS, polyoxylglycerides such as labrafil, gelucire 44/14, labrasol; polyethoxylated castor oil such as cremophore or any combinations thereof.

Solvents include but are not limited to water, dipolar aprotic solvent, polyethylene glycol, polyethyleneglycol ether, polyethyleneglycol derivative of a mono- or di-glyceride, buffers, water soluble organic solvents, organic solvents or any combination thereof.

The surfactants include but are not limited to polyoxylglycerides such as labrafil, gelucire 44/14, Labrasol; polyethoxylated castor oil such as Cremophor RH40, Cremophor ELP, Polysorbate 80 HP or Vitamin E TPGS and the like, or any combination thereof.

Oils include but are not limited to a medium chain triglyceride, castor oil, a medium chain mono-glyceride, a medium chain di-glyceride, an edible vegetable oil such as peanut oil, cottonseed oil, or soybean oil, or any combination thereof. Alternatively, the oil can be other than a glyceride; for example, the oil can be a hydrocarbon oil or a silicone oil and the like or any combinations thereof.

The emulsifiers include but are not limited to lipids and phospholipids such as lecithin. The lecithin can be a high phosphatidylcholine content lecithin, a low phosphatidylcholine content lecithin and the like or any combination thereof.

Stabilizing agents include but are not limited to antioxidants, alkalizer, pH modifier, substances preventing crystallization or any combinations thereof.

Solid dispersion of rifaximin comprises rifaximin from about 0.1% to 90% by weight of the total weight of solid dispersion. The therapeutic dose varies according to the body weight and the acuteness of the pathology; a daily dose between upto 2400 mg, administered in a single dose or divided into 2 or 3 or more doses.

The foregoing examples are illustrative embodiments of the invention and are merely exemplary.

| **Ingredients** | **Examples** | | | | | | | |
|---|---|---|---|---|---|---|---|---|
| | **1** | **2** | **3** | **4** | **5** | **6** | **7** | **8** |
| Rifaximin | 600 g | 100 g | 100g | 600 g | 600 g | 600 g | 600 g | 100 g |
| Povidone | 600 g | 200 g | 300g | | | 350 g | 400 g | |
| Co-Povidone | | | | | 500 g | | | |
| Poloxamer | | 100 g | | | | | | |
| Polyethylene glycol | | | | | 20 g | | | 300g |
| α TPGS | | | | 400 g | | | | |
| Tween 80 | | | | | | 50 g | | |
| Span 20 | | | | | | | 45 g | |
| Alcohols | | q.s. | q.s. | | | | q.s. | q.s. |
| Water | | | | | | | q.s. | |

The above examples are prepared by following methods.

### Example 1: Solid dispersion by fusion

### Procedure:

1. Sift all ingredients through suitable sieve
2. Melt the weighed quantity of povidone
3. Add rifaximin to the above melted portion of povidone with stirring.
4. Cool the mass at room temperature then mill and pass through desired sieve.

### Examples 2: Solid dispersion by solvent evaporation technique

### Procedure:

1. Sift all ingredients through suitable sieve
2. Dissolve rifaximin, povidone and poloxamer in alcohol with stirring.
3. Evaporate the above mixture
4. Scrap the above mass then mill and pass through desired sieve.

### Examples 3: Solid dispersion by solvent evaporation technique

### Procedure:

1. Sift all ingredients through suitable sieve
2. Dissolve rifaximin and povidone in alcohol with stirring.
3. Evaporate the above mixture
4. Scrap the above mass then mill and pass through desired sieve.

### Example 4: Solid dispersion by using hot melt extrusion technique by using watersoluble polymers

### Procedure:

1. Sift all ingredients through suitable sieve
2. Mix all the sifted materials uniformly.
3. Add the above mixture in a hot melt extruder and collect the extrudate
4. Mill the above extrudate and pass through desired sieve.

### Example 5: Solid dispersion by using hot melt extrusion technique by using watersoluble polymers and plasticizer

### Procedure:

1. Sift all ingredients through suitable sieve
2. Mix all the sifted materials uniformly.
3. Add the above mixture in a Hot Melt Extruder and collect the extrudate
4. Mill the above extrudate and pass through desired sieve

### Example 6: Solid dispersion by using hot melt extrusion technique by using watersoluble polymers and surfactant

### Procedure:

1. Sift all ingredients through suitable sieve
2. Mix all the sifted materials uniformly.
3. Add the above mixture in a hot melt extruder and collect the extrudate.
4. Mill the above extrudate and pass through desired sieve

### Example 7: Solid dispersion by using co-precipitation technique

### Procedure:

1. Sift all ingredients through suitable sieve
2. Dissolve weighed quantity of rifaximin, povidone and Span 20 in alcohol with stirring.
3. Add water to above solution with stirring to form a precipitate.
4. Dry the precipitate, mill and pass through the desired sieve.

### Example 8: Solid dispersion by using solvent evaporation technique

### Procedure:

1. Sift all ingredients through suitable sieve
2. Dissolve weighed quantity of rifaximin and polyethylene glycol in alcohol with stirring.
3. evaporate the above mixtures by using rotary evaporator.
4. scrap the above mass then mill and pass through desired sieve.

The solid dispersion of rifaximin provides higher solubility of rifaximin and improves gastrointestinal (GIT) availability relative to an equivalent dose of rifaximin in a commercially available tablet or dosage form, or to an equivalent dose of rifaximin in a simple solution such as in water or normal saline solution or gastrointestinal simulated fluid or buffer, that is orally ingested. The increase in solubility is more than 30%.

Also disclosed are various methods for screening and analyzing methods and preparations that are intended to enhance the dissolution of poorly soluble drugs. These analysis methods include dissolution studies, saturation solubility studies, wettability studies, permeation studies, X-ray diffraction studies etc.

Solubility study reveals that the solubility of pure rifaximin in pH 6.8 Tris Phosphate buffer and in purified water is 0.01 mg/ml and less than 10µg/ml respectively whereas the solubility of solid dispersion prepared by using rifaximin, polaxamer 188 and povidone (1:1:2) in pH 6.8 Tris Phosphate buffer and purified water is 0.23 mg/ml and 0.38 mg/ml respectively. Likewise the solubility of solid dispersion prepared by using rifaximin and povidone (1:3) in pH 6.8 Tris Phosphate buffer and purified water is 0.35 mg/ml and 0.38 mg/ml respectively. Hence, there is an increase in the solubility of solid dispersion of rifaximin which leads to improved gastrointestinal availability i.e. without increase in systemic absorption which is evident from the cell permeation study.

### Permeability study for rifaximin and solid dispersion of rifaximin using MDCK (Mardin Darby Canine Kidney cells) Cells:

Place 10µmolar of the final concentration of rifaximin and solid dispersion of rifaximin in the apical side of the transwell plates cultured with MDCK cells. Three well are to be used per sample. Plates are placed in shaking water bath at 37°C for 2 hours. Take out 20µl from apical and 100µl from basolateral side for sample processing at 0 and 180 min. Lucifer Yellow is used as a marker to see the monolayer integrity of the cells. Propronalol is used as a permeability marker.

The integrity of the MDCK cells was found to be suitable as adjusted by Lucifer Yellow. Relative area in apical remain 100% even after 2 hours. No detectable amount of rifaximin was found in the basolateral side of the transwell, thereby reflecting towards no apparent permeability (since the basal value is 0 we could not calculate the P_{APP} through equations).

Solid dispersion and the compositions containing solid dispersion of rifaximin are advantageous as rifaximin is a locally acting antibiotic; hence higher intestinal levels can be achieved. Further, the dose of rifaximin generally used for treatment of various pathogenic diseases may be reduced, as the minimum inhibitory concentration (in vitro) of rifaximin for various organisms is very less than the concentration of rifaximin in the feces. After oral administration of 400 mg 14C-rifaximin to healthy volunteers, approximately 97% of the dose was recovered in feces, almost entirely as unchanged drug, and 0.32% was recovered in the urine.

Minimum inhibitory concentration (MIC) is defined as the lowest concentration of rifaximin that completely inhibits visible growth (a fine, barely visible haze or a single colony will be disregarded).

**Table 1: MIC90 of rifaximin for selected enteric pathogens**

| Pathogen | No. of Strains | MIC₉₀ µg/ml |
|---|---|---|
| Campylobacter jejuni | 9 54 | 32 128 |
| Clostridium difficile | 93 | 128 |
| | 56 | 128 |
| Escherichia coli | | |
| Enteroaggregative | 75 | 32 |
| Enterotoxigenic | 97 | 32 |
| Helicobacter pylori | 40 | 4 (MIC₅₀) |
| Salmonella species | 46 | 64 |
| Shigella species | 36 | 64 |
| Vibrio cholerae | 403 | 4 |
| Yersinia enterocolitica | 74 | 16 |
| Other | 21 | 4 |

The dose of rifaximin in form of solid dispersion can be reduced as its solubility is increased. A quantitative method (diffusion technique) is used to evaluate the antimicrobial effect of solid dispersion of rifaximin with respect to pure rifaximin. The measurement of zone diameters estimates the susceptibility of bacteria to antimicrobial compounds.

### Determination of anti-microbial activity of rifaximin and solid dispersion of rifaximin by using cup plate method or well plate method:

Prepare a nutrient agar medium in distilled water and sterilize this media in autoclave at 121°C for 15 min. Transfer this media aseptically in sterile petriplate, allow the media to solidify for few minutes. After solidification, add test micro-organism i.e. E. Coli (100 µl) in each plate and spread this culture with the help of spreader. Prepare the well in plate with the help of borer. Add the test sample (rifaximin and solid dispersion of rifaximin) 200 µl in each well aseptically; incubate this plate in an incubator at 37°C for 24 hrs and then observe the zone of inhibition around the well.

Rifaximin after 24hrs shows very negligible area of zone of inhibition where as solid dispersion rifaximin with povidone (1:3) and solid dispersion rifaximin with poloxamer and povidone (1:1:2) shows area of zone of inhibition 15mm and 10 mm respectively.

Solid dispersion of rifaximin of the invention can be formulated into solid, semi-solid or liquid preparations along with pharmaceutically acceptable excipient(s), into tablets (single layered tablets, multilayered tablets, mini tablets, bioadhesive tablets, caplets, matrix tablets, tablet within a tablet, mucoadhesive tablets, modified release tablets, pulsatile release tablets, timed release tablets, delayed release, controlled release, extended release and sustained release tablets), pellets, beads, granules, sustained release compositions, pills, troches, capsules hard and soft or liquid filled soft gelatin capsules, microcapsules, minitablets, tablets in capsules and microspheres, matrix compositions, osmotic compositions, bioadhesive compsoitions, powder/pellets/granules for suspension, powder, solutions, suspensions, sachets or emulsions and the like.

The composition comprising solid dispersion of rifaximin may comprise pharmaceutically acceptable excipients include but are not limited to binders, diluents, lubricants, glidants and surface-active agents.

The amount of additive employed will depend upon how much active agent is to be used. One excipient can perform more than one function.

Binders include, but are not limited to, starches such as potato starch, wheat starch, corn starch; microcrystalline cellulose; celluloses such as hydroxypropyl cellulose, hydroxyethyl cellulose, hydroxypropylmethyl cellulose (HPMC), ethyl cellulose, sodium carboxy methyl cellulose; natural gums like acacia, alginic acid, guar gum; liquid glucose, dextrin, povidone, syrup, polyethylene oxide, polyvinyl pyrrolidone, poly-N-vinyl amide, polyethylene glycol, gelatin, poly propylene glycol, tragacanth, combinations there of and other materials known to one of ordinary skill in the art and mixtures thereof.

Fillers or diluents include, but are not limited to, confectioner's sugar, compressible sugar, dextrates, dextrin, dextrose, fructose, lactitol, mannitol, sucrose, starch, lactose, xylitol, sorbitol, talc, microcrystalline cellulose, calcium carbonate, calcium phosphate dibasic or tribasic, calcium sulphate, and the like can be used.

Lubricants may be selected from, but are not limited to, those conventionally known in the art such as Magnesium, Aluminium or Calcium or Zinc stearate, polyethylene glycol, glyceryl behenate, mineral oil, sodium stearyl fumarate, stearic acid, hydrogenated vegetable oil and talc.

Glidants include, but are not limited to silicon dioxide, magnesium trisilicate, powdered cellulose, starch, talc and tribasic calcium phosphate, calcium silicate, magnesium silicate, colloidal silicon dioxide, silicon hydrogel and other materials known to one of ordinary skill in the art.

The compositions comprising solid dispersion of rifaximin may optionally contain a surface-active agent. The preferred surface-active agent is copolymers composed of a central hydrophobic chain of polyoxypropylene (poly (propylene oxide)) and polyoxyethylene (poly (ethylene oxide)) that is well known as poloxamer. However, other surface-active agents may also be employed such as dioctyl sodium sulfosuccinate (DSS), triethanolamine, sodium lauryl sulphate (SLS), polyoxyethylene sorbitan and poloxalkol derivatives, quaternary ammonium salts or other pharmaceutically acceptable surface-active agents known to one ordinary skilled in the art.

Also disclosed are modified release pharmaceutical compositions comprising solid dispersion of rifaximin for oral use.

Alos disclosed are various combinations of immediate release composition comprising solid dispersion of rifaximin and modified release composition comprising solid dispersion of rifaximin.

The pharmaceutical dosage form comprising solid dispersion of rifaximin can optionally have one or more coatings such as film coating, sugar coating, enteric coating, bioadhesive coating and other coatings known in the art. These coatings help pharmaceutical compositions to release the drug at the required site of action. In one example, the additional coating prevents the dosage from contacting the mouth or esophagus. In another example, the additional coating remains intact until reaching the small intestine (e.g., an enteric coating). Premature exposure of a bioadhesive layer or dissolution of a pharmaceutical dosage form in the mouth can be prevented with a layer or coating of hydrophilic polymers such as HPMC or gelatin. Optionally, Eudragit FS 30D or other suitable polymer or agent may be incorporated in coating composition to retard the release of the drug to ensure drug release in the colon.

These coating layers comprises one or more excipients selected from the group comprising coating agents, opacifiers, taste-masking agents, fillers, polishing agents, colouring agents, antitacking agents and the like.

Coating agents which are useful in the coating process, include, but are not limited to, polysaccharides such as maltodextrin, alkyl celluloses such as methyl or ethyl cellulose, hydroxyalkylcelluloses (e.g. hydroxypropylcellulose or hydroxypropylmethylcelluloses); polyvinylpyrrolidone, acacia, corn, sucrose, gelatin, shellac, cellulose acetate pthalate, lipids, synthetic resins, acrylic polymers, opadry, polyvinyl alcohol (PVA), copolymers of vinylpyrrolidone and vinyl acetate (e.g. marketed under the brand name of Plasdone) and polymers based on methacrylic acid such as those marketed under the brand name of Eudragit. These may be applied from aqueous or non-aqueous systems or combinations of aqueous and non-aqueous systems as appropriate. Additives can be included along with the film formers to obtain satisfactory films. These additives can include plasticizers such as dibutyl phthalate, triethyl citrate, polyethylene glycol (PEG) and the like, antitacking agents such as talc, stearic acid, magnesium stearate and colloidal silicon dioxide and the like, surfactants such as polysorbates and sodium lauryl sulphate, fillers such as talc, precipitated calcium carbonate, Polishing agents such as beeswax, carnauba wax, synthetic chlorinated wax and opacifying agents such as titanium dioxide and the like. All these excipients can be used at levels well known to the persons skilled in the art.

Pharmaceutical dosage forms comprising solid dispersion of rifaximin can be coated by various methods. Suitable methods include compression coating, coating in a fluidized bed or a pan and hot melt (extrusion) coating. Such methods are well known to those skilled in the art.

Non-permeable coatings of insoluble polymers, e.g., cellulose acetate, ethylcellulose, can be used as enteric coatings for delayed/modified release (DR/MR) by inclusion of soluble pore formers in the coating, e.g., PEG, PVA, sugars, salts, detergents, triethyl citrate, triacetin, etc.

Also, coatings of polymers that are susceptible to enzymatic cleavage by colonic bacteria are another means of ensuring release to distal ileum and ascending colon. Materials such as calcium pectinate can be applied as coatings to dosage form and multiparticulates and disintegrate in the lower gastrointestinal tract, due to bacterial action. Calcium pectinate capsules for encapsulation of bioadhesive multiparticulates are also available.

The coating may further comprise the drug.

Also disclosed is apharmaceutical composition comprising solid dispersion of rifaximin is multilayer tablets comprising a first, a second and/or a third layer, where each layer includes one or more excipient(s). Multi-layer or gradient tablets can be assembled in several different ways.

In one example, the tablet comprising solid dispersion of rifaximin comprises at least one solid core and two outer layers, comprising one or more pharmaceutical excipients. The core comprises solid dispersion of rifaximin and release controlling agents. The two outer layers are bioadhesive.

In another example, the tablet comprising solid dispersion of rifaximin comprises at least one core and two outer layers, comprising drug and one or more pharmaceutical excipients. Such tablets can also be used to commence release of different drugs at different times, by inclusion of different drugs in separate layers.

In another example, the multi-layer tablet comprising solid dispersion of rifaximin comprises of a core and two outer layers, comprising solid dispersion of rifaximin and one or more pharmaceutical excipients, wherein at least one excipient is hydrophobic.
Another embodiment relates to composition comprising solid dispersion of rifaximin, which comprises multilayer tablet wherein atleast one layer consist of one or more release controlling agents and solid dispersion of rifaximin and at least one layer which consist of bioadhesive agents, where each layer includes one or more excipients.

Also disclosed is a composition comprising solid dispersion of rifaximin, which comprises multilayer tablet wherein atleast one layer consist of one or more release controlling agents and at least one layer which consist of bioadhesive agent, where each layer includes one or more excipients and solid dispersion of rifaximin.

The release controlling agents can be hydrophilic or hydrophobic or combination thereof.

The hydrophilic rate-controlling agents are selected from, but are not limited to hydroxyethylcellulose, hydroxypropyl cellulose, hydroxypropyl methylcellulose (e.g. hypromellose), sodium carboxymethyl cellulose, sodium alginate, carbomer (Carbopol™), xanthan gum, guar gum, locust bean gum, poly vinyl acetate, polyvinyl alcohol or combinations thereof.

The hydrophobic rate controlling agents in matrix includes but are not limited to hydrogenated vegetable oil, but other suitable agents include purified grades of beeswax; fatty acids; long chain fatty alcohols, such as cetyl alcohol, myristyl alcohol, and stearyl alcohol; glycerides such as glyceryl esters of fatty acids like glyceryl monostearate, glyceryl distearate, glyceryl esters of hydrogenated castor oil and the like; oils such as mineral oil and the like, or acetylated glycerides; ethyl cellulose, stearic acid, paraffin, carnauba wax, talc; and the stearate salts such as calcium, magnesium, zinc and other materials known to one of ordinary skill in the art or combinations thereof.

Also disclosed is a pharmaceutical composition comprising solid dispersion of rifaximin and atleast one swellable polymer. Swellable polymers include, but are not limited to, a crosslinked poly (acrylic acid), a poly (alkylene oxide), a polyvinyl alcohol), a polyvinyl pyrrolidone); a polyurethane hydrogel, a maleic anhydride polymer, such as a maleic anhydride copolymer, a cellulose polymer, a polysaccharide, starch, and starch based polymers.

The pharmaceutical composition comprising solid dispersion of rifaximin can be prepared by various methods known in the art such as by dry granulation, wet granulation, melt granulation, direct compression, double compression, extrusion spheronization, layering and the like.

The pharmaceutical composition comprising solid dispersion of rifaximin of the invention will be hereinafter described in more detail by referring to examples given below but only for purposes of illustration and the invention is not limited by them.

### Example 9

| **Rifaximin 200 mg tablet** | |
|---|---|
| **Ingredients** | **mg/tablet** |
| Solid Dispersion Rifaximin | 800 |
| Microcrystalline Cellulose | 117 |
| Sodium starch glycolate | 38 |
| Colloidal silicon di-oxide | 20 |
| Mg-stearate | 25 |
| Average weight of tablet | 1000 |

The solid dispersion of rifaximin, microcrystalline cellulose, half a quantity each of sodium starch glycolate, colloidal silicon di-oxide and mg-stearate are sieved, mixed uniformly and then slugged using roller compactor. The slugs are de-slugged and mixed with remaining quantity of sodium starch glycolate, colloidal silicon di-oxide and mg-stearate and then compressed using suitable punch.

### Example 10

| **Rifaximin 150 mg MR tablet** | |
|---|---|
| **Ingredients** | **mg/tablet** |
| Solid Dispersion Rifaximin | 600 |
| Hypromellose | 100 |
| Mannitol | 40 |
| Colloidal silicon di-oxide | 20 |
| Mg-stearate | 25 |
| Average weight of tablet | 785 |

The solid dispersion of rifaximin, mannitol, hypromellose and half a quantity each of colloidal silicon di-oxide and mg-stearate are sieved, mixed uniformly and then slugged using roller compactor. The slugs are de-slugged and mixed with remaining quantity of colloidal silicon di-oxide and mg-stearate and then compressed using suitable punch.

### Example 11

| **Rifaximin 200 mg ER tablet** | |
|---|---|
| **Ingredients** | **mg/tablet** |
| First layer | |
| Solid Dispersion Rifaximin | 800 |
| Hypromellose | 100 |
| Mannitol | 40 |
| Microcrystalline Cellulose | 30 |
| Colloidal silicon di-oxide | 20 |
| Mg-stearate | 25 |
| Average weight of First layer | 1015 |

| Second layer (Bio-adhesive layer) | |
|---|---|
| Polyethylene Oxide | 90 |
| Hypromellose | 90 |
| Colloidal silicon di-oxide | 20 |
| Mg-stearate | 20 |
| Average weight of Second layer | 220 |
| Total weight of Bi-layer tablet | 1235 |

The solid dispersion of rifaximin, mannitol, hypromellose, microcrystalline cellulose and half a quantity each of colloidal silicon di-oxide and mg-stearate are sieved, mixed uniformly and then slugged using roller compactor. The slugs are de-slugged and mixed with remaining quantity of colloidal silicon di-oxide and mg-stearate and then compressed using suitable punch. Polyethylene oxide and hypromellose, colloidal silicon di-oxide and mg-stearate of second layer are sieved, mixed uniformly and compressed with the drug layer by suitable punch.

Another embodiment relates to the effect of the solubility of rifaximin on various enteric pathogens or bacterial susceptibility and subsequent eradication.

Another object of the invention is to provide a cost effective composition of rifaximin or a pharmaceutically acceptable salt.

Another embodiment relates to the effect of the solubility of rifaximin on various pathogens or bacterial susceptibility and subsequent eradication due to the invasive nature of some enteric pathogens.

Another example relates to the effect of the solubility of rifaximin on the development of resistance to various pathogens or bacterial susceptibility.

Also disclosed is a pharmaceutical composition comprising stable solid dispersion comprising rifaximin for treatment of traveler's diarrhea, hepatic encephalopathy, infectious diarrhea, diverticular disease, an antibacterial prophylactic prior to colon surgery, irritable bowel syndrome, inflammatory bowel disease, Crohn's disease, Clostridum difficile-associated diarrhea, small intestinal bacterial overgrowth, traveler's diarrhea prophylaxis, dysentery, pouchitis, peptic ulcer disease, surgical prophylaxis and gastric dyspepsia. Further, it can be used in treating Klebsiella caused traveler's diarrhea. It can be administered in various dosing regimens such as repeatedly or once at a dose, in a frequency, and for a duration sufficient to provide a beneficial effect to the patient.

A rifaximin composition comprising solid dispersion of rifaximin can be administered in conjunction with a second pharmaceutically active agent such as antibiotics, antimotility agents, anti-inflammatory agents, anti-infectives, diuretics, anti-diabetics, anti-fungal, analgesics, antiallergics, anti-ulceratives, antiemetics, immunosupressants, antivirals, antiretrovirals, acidity reducing agents, steroids, enzymes, enzyme inhibitors, proteins, peptides and antitussives or combinations thereof. The second pharmaceutically active agent can be included in the oral composition and thus administered in a combination with rifaximin, or can be administered separately. If administered separately, it can be administered substantially concurrently, prior to, or after administration of the oral composition. The second pharmaceutically active agent can be administered orally or parenterally, for example intravenously. The second pharmaceutically active agent can be provided at doses, frequencies of administration, and over duration of time in combination with rifaximin doses, frequencies of administration, and over duration of time effective to provide a beneficial effect to the patient.

A composition comprising solid dispersion of rifaximin may be provided as a kit; i.e., enclosed in packaging with instruction materials.

## Claims

1. A solid dispersion of rifaximin comprising rifaximin and pharmaceutically acceptable carrier, wherein the pharmaceutically acceptable carrier is selected from polyethylene glycol, polymethacrylates, polyvinyl acetate, cellulose derivatives, polaxamer, glyceryl behenate, polyethyleneglycol derivative of a mono-glyceride, vitamin E, polyethylene or polyoxyethylene ester of hydroxyl stearic acid, polyoxylglycerides, polyethoxylated castor oil or combinations thereof,
wherein the solubility of rifaximin is increased relative to an equivalent amount of rifaximin not in solid dispersion.

2. The solid dispersion of claim 1, wherein the gastrointestinal availability of rifaximin is increased relative to an equivalent amount of rifaximin.

3. The solid dispersion of rifaximin of claim 1 wherein the solubility of rifaximin in the solid dispersion is increased relative to an equivalent amount of rifaximin, while maintaining equivalent permeability relative to an equivalent amount of rifaximin.

4. A pharmaceutical composition comprising solid dispersion of claim 1.

5. A pharmaceutical composition comprising the solid dispersion of claim 1 for use in the treatment and/or prevention of a microbial infection.

6. A pharmaceutical composition comprising the solid dispersion of claim 1 for use in the treatment and/or prevention of irritable bowel syndrome or Crohn's disease.

7. The solid dispersion of claim 1, wherein the dispersion provides higher solubility of rifaximin and improved gastrointestinal (GIT) availability relative to an equivalent dose of rifaximin in a commercially available tablet, or to an equivalent dose of rifaximin in a simple solution such as in water or normal saline solution, that is orally ingested.

8. The solid dispersion of claim 1, wherein the solubility of rifaximin is increased by more than 30%.

## Patentansprüche

1. Feste Dispersion von Rifaximin, umfassend Rifaximin und einen pharmazeutisch annehmbaren Träger, wobei der pharmazeutisch annehmbare Träger ausgewählt ist aus Polyethylenglykol, Polymethacrylaten, Polyvinylacetat, Cellulosederivaten, Polaxamer, Glycerylbehenat, Polyethylenglykolderivat eines Monoglycerids, Vitamin E, Polyethylen oder Polyoxyethylenester von Hydroxylstearinsäure, Polyoxylglyceriden, polyethoxyliertem Rizinusöl oder Kombinationen davon,
wobei die Löslichkeit von Rifaximin in Bezug auf eine entsprechende Menge von Rifaximin nicht in fester Dispersion erhöht ist.

2. Feste Dispersion nach Anspruch 1, wobei die gastrointestinale Verfügbarkeit von Rifaximin im Verhältnis zu einer entsprechenden Menge von Rifaximin erhöht ist.

3. Feste Dispersion von Rifaximin nach Anspruch 1, wobei die Löslichkeit von Rifaximin in der festen Dispersion in Bezug auf eine entsprechende Menge von Rifaximin erhöht ist, während eine entsprechende Permeabilität in Bezug auf eine entsprechende Menge von Rifaximin beibehalten wird.

4. Pharmazeutische Zusammensetzung, umfassend eine feste Dispersion nach Anspruch 1.

5. Pharmazeutische Zusammensetzung, umfassend die feste Dispersion nach Anspruch 1 zur Verwendung in der Behandlung und/oder Prävention einer mikrobiellen Infektion.

6. Pharmazeutische Zusammensetzung, umfassend die feste Dispersion nach Anspruch 1 zur Verwendung in der Behandlung und/oder Prävention von Reizdarmsyndrom oder Morbus Crohn.

7. Feste Dispersion nach Anspruch 1, wobei die Dispersion eine höhere Löslichkeit von Rifaximin und eine verbesserte gastrointestinale (GIT) Verfügbarkeit in Bezug auf eine entsprechende Dosis von Rifaximin in einer handelsüblichen Tablette oder auf eine entsprechende Dosis von Rifaximin in einer einfachen Lösung, wie beispielsweise in Wasser oder einer normalen Salzlösung, die oral eingenommen wird, bereitstellt.

8. Feste Dispersion nach Anspruch 1, wobei die Löslichkeit von Rifaximin um mehr als 30 % erhöht ist.

## Revendications

1. Dispersion solide de rifaximine comprenant de la rifaximine et un support pharmaceutiquement acceptable, dans laquelle le support pharmaceutiquement acceptable est sélectionné parmi le polyéthylène glycol, des polyméthacrylates, l'acétate de polyvinyle, des dérivés de la cellulose, un poloxamère, le béhénate de glycéryle, un dérivé de polyéthylèneglycol d'un mono-glycéride, la vitamine E, un ester de polyéthylène ou polyoxyéthylène d'acide hydroxystéarique, des polyoxylglycérides, l'huile de ricin polyéthoxylée ou des combinaisons de ceux-ci, dans laquelle la solubilité de la rifaximine est augmentée par rapport à une quantité équivalente de rifaximine qui n'est pas dans une dispersion solide.

2. Dispersion solide selon la revendication 1, dans laquelle la disponibilité gastro-intestinale de la rifaximine est augmentée par rapport à une quantité équivalente de rifaximine.

3. Dispersion solide de rifaximine selon la revendication 1 dans laquelle la solubilité de la rifaximine dans la dispersion solide est augmentée par rapport à une quantité équivalente de rifaximine, tout en maintenant une perméabilité équivalente par rapport à une quantité équivalente de rifaximine.

4. Composition pharmaceutique comprenant la dispersion solide selon la revendication 1.

5. Composition pharmaceutique comprenant la dispersion solide selon la revendication 1 destinée à être utilisée dans le traitement et/ou la prévention d'une infection microbienne.

6. Composition pharmaceutique comprenant la dispersion solide selon la revendication 1 destinée à être utilisée dans le traitement et/ou la prévention du syndrome du côlon irritable ou de la maladie de Crohn.

7. Dispersion solide selon la revendication 1, dans laquelle la dispersion fournit une plus haute solubilité de rifaximine et une disponibilité gastro-intestinale (GIT) améliorée par rapport à une dose équivalente de rifaximine dans un comprimé disponible dans le commerce, ou à une dose équivalente de rifaximine dans une solution simple telle que dans l'eau ou une solution saline normale, qui est ingérée par voie orale.

8. Dispersion solide selon la revendication 1, dans laquelle la solubilité de la rifaximine est augmentée de plus de 30 %.
